# DEMANDE DE BREVET EUROPEEN

(11) **EP 0 570 255 A1**
(43) Date de publication de la demande: **18.11.1993**
(21) Numéro de dépôt: 93401124.8
(22) Date de dépôt: 29.04.1993
(51) Int. Cl.: A61C 17/06, A61C 1/16

(54) **Dispositif jetable pour la protection d'une unité d'aspiration chirugicale, notamment pour cabinets dentaires**

(30) Priorité: 29.04.1992 FR 9205293
(71) Demandeur: BAILLY, Alain, F-91700 Sainte-Geneviève-des-Bois (FR); Michaux, Guy, F-75007 Paris (FR)
(72) Inventeur: Bailly, Alain, F-91700 Sainte-Geneviève des Bois (FR)
(74) Mandataire: Sauvage, Renée

(57) **Abrégé**

L'invention est applicable aux unités du type comprenant un conduit d'aspiration (4) ayant une extrémité amont comportant un embout (1) recevant une canule d'aspiration (7) et une extrémité aval reliée à une source de vide.

Le dispositif comprend : d'une part, un adaptateur (8) dont l'extrémité amont est adaptée à être connectée avec ladite canule (7) et dont l'extrémité aval est adaptée à être connectée avec l'extrémité amont (2) dudit embout (1) et, d'autre part, une housse tubulaire (16) dont une extrémité est solidarisée audit adaptateur (8) et dont l'autre extrémité est libre, ladite housse définissant une cavité adaptée à recevoir ledit embout (1) et l'extrémité amont dudit conduit (4) lorsque ledit embout (1) est connecté audit adaptateur (8).

## Description

La présente invention concerne un dispositif jetable pour la protection d'une unité d'aspiration chirurgicale, notamment pour cabinets dentaires.

Les unités d'aspiration chirurgicale ont un conduit d'aspiration ayant une extrémité amont et une extrémité aval, par référence au sens d'écoulement sous l'effet de l'aspiration, ladite extrémité amont comportant un embout adapté à recevoir une canule d'aspiration et ladite extrémité aval étant susceptible d'être reliée à une source de vide.

Ces embouts et conduits présentent des aspérités et des canelures qui les rendent très difficiles à nettoyer et à décontaminer. Par suite, même si l'environnement est parfaitement aseptique, les unités d'aspiration chirurgicale constituent un risque important d'infection et de contamination.

La présente invention a pour but de remédier à cette situation et, à cet effet, elle propose un dispositif qui est caractérisé en ce qu'il comprend :
- d'une part, un adaptateur présentant une extrémité amont et une extrémité aval mises en communication par un canal traversant, ladite extrémité amont de l'adaptateur étant adaptée à être connectée avec ladite canule, tandis que son extrémité aval est adaptée à être connectée avec l'extrémité amont dudit embout et,
- d'autre part, une housse tubulaire dont une extrémité est solidarisée audit adaptateur et dont l'autre extrémité est libre, ladite housse définissant une cavité adaptée à recevoir ledit embout et l'extrémité amont dudit conduit lorsque ledit embout est connecté audit adaptateur.

On comprend que l'adaptateur est ainsi interposé entre la canule et l'unité d'aspiration et que la paroi extérieure de l'embout et du conduit est protégée des inévitables projections par la housse.

Dans une forme de réalisation pratique de l'invention, l'adapteur est connectable, d'une part, à ladite canule par réception de l'extrémité de ladite canule dans l'extrémité amont dudit canal et, d'autre part, audit embout par réception de son extrémité aval dans l'extrémité amont dudit embout.

L'adaptateur et son canal traversant sont de section circulaire et, en général, le diamètre interne de l'extrémité amont dudit canal traversant sera égal au diamètre externe aval dudit adaptateur.

Le retour vers le site chirurgical des liquides corporels et débris aspirés devant être évité, l'extrémité aval dudit canal est munie d'un clapet anti-retour qui, dans une forme d'exécution préférée, peut être un clapet souple à lèvres normalement jointives mais adaptées à s'écarter sous l'effet d'une aspiration. Ce clapet peut par exemple être du type "sifflet" ou avoir des lèvres en croix.

En pratique, l'adaptateur est en matière plastique moulée et la housse est en un film de matière plastique transparent, ne gênant ainsi aucunement ni la manipulation de l'unité par le praticien, ni la mise en place de l'adaptateur sur ou dans l'embout.

Avant utilisation, le dispositif selon l'invention est conditionné dans un emballage étanche et au contenu stérilisé. Après utilisation, il est séparé de l'unité d'aspiration et jeté avec la canule, si l'on a utilisé une canule jetable, ou après séparation d'avec la canule dans le cas contraire.

L'invention est décrite en détail ci-après par référence aux dessins annexés dans lesquels :
- les figures 1a à 1c sont des vues en bout, côté extrémité amont, de trois formes d'exécution de l'invention,
- les figures 2a à 2c sont des vues en coupe respectivement des adaptateurs des figures 1a à 1c, et
- la figure 3 est une vue en perspective montrant le dispositif selon l'invention en place sur une unité d'aspiration.

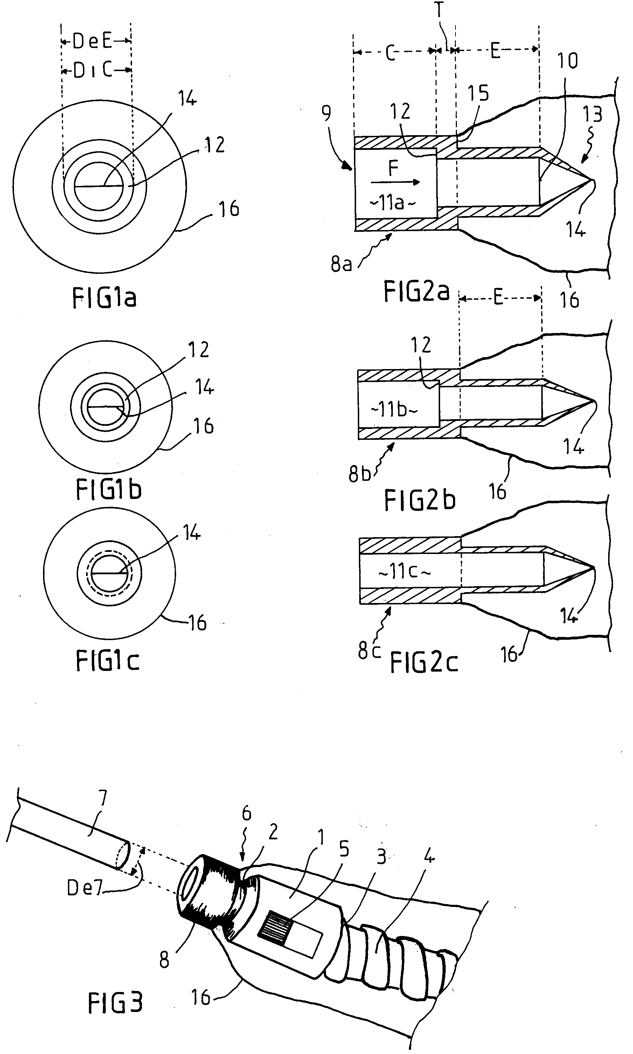

Si l'on se réfère tout d'abord à la figure 3, on voit l'extrémité amont d'une unité d'aspiration comportant un embout 1 dont l'extrémité amont 2 est adaptée à recevoir directement une canule d'aspiration, schématisée en 7, et dont l'extrémité aval 3 est connectée à un conduit 4 du type "flexible" lui-même relié à une source de vide (non représentée). La paroi périphérique externe de l'embout 1 présente une fenêtre donnant accès à une commande 5 agissant sur un clapet de mise en, ou hors de, communication de l'extrémité amont 2 de l'embout 1 avec la source de vide. La structure décrite jusqu'ici est classique.

On comprend qu'il est extrêmement difficile de garantir la propreté de l'embout 1 et du conduit 4 et de les décontaminer du fait qu'ils présentent des reliefs et des zones pratiquement inaccessibles. C'est le cas en particulier des articulations du conduit flexible.

Selon l'invention, un dispositif protecteur 6 isole l'embout 1 et la partie la plus amont du conduit 4 des projections et souillures diverses. Ce dispositif protecteur comprend, d'une part, un adaptateur 8 interposé entre l'embout 1 et la canule 7 et, d'autre part, une housse 16 solidarisée à l'adaptateur 8 et venant recouvrir l'embout 1 et la partie voisine du conduit 4.

La structure du dispositif 6 ressort mieux des figures 1a-2a, 1b-2b et 1c-2c.

Comme le montrent les figures 1a et 2a, l'adaptateur 8a est constitué d'une pièce ayant une extrémité amont 9 et une extrémité aval 10̸, par référence au sens de l'écoulement sous l'effet de l'aspiration (flèche F). L'adaptateur 8a, de section transversale circulaire, est creux et il définit un canal traversant 11a, également de section circulaire, mettant en communication ses extrémités amont et aval. L'adaptateur 8a comporte une zone C de connexion à une canule, telle que 7, une zone E de connexion à un embout, tel que 1, et une zone de transition T entre les zones C et E. Le diamètre interne DiC de la zone C, et donc de l'extrémité amont 9 du canal 11a correspond à un diamètre standard externe De7 (figure 3), par exemple 16 mm, de canule. Le diamètre externe DeE de la zone E, donc de l'extrémité aval de l'adaptateur, correspond au diamètre interne de l'embout 1 adapté à recevoir ladite canule standard, donc également à 16 mm dans l'exemple pris. L'égalité entre le diamètre interne amont DiC et le diamètre externe aval DeE implique une réduction de diamètre et, par suite, la présence d'une paire d'épaulements annulaires interne 12 et externe 15. L'épaulement 12 forme butée lors de l'introduction de la canule dans la zone C et l'épaulement 15 forme butée lors de l'introduction de la zone E dans l'embout 1.

L'extrémité aval 10̸ de l'adaptateur est pourvue d'un clapet en sifflet 13 dont les deux lèvres se rejoignent selon une fente 14. Ces lèvres s'ouvrent sous l'effet d'une aspiration selon la flèche F, mais se tiennent fermées en l'absence d'aspiration, s'opposant à tout retour de fluide ou de débris aspiré, vers la canule.

Sur l'épaulement 15 est fixée, par exemple soudée, une housse tubulaire 16 en mince film plastique transparent et souple, par exemple de 5/10̸0̸ mm d'épaisseur. On comprend que, pour la mise en place du dispositif selon l'invention sur l'unité d'aspiration, on enfile la housse 16 sur l'embout 1 et la partie adjacente du conduit 4 et que l'on introduit ensuite la zone E de l'adaptateur 8a dans l'extrémité amont 2 de l'embout 1. La transparence du film permet de voir sans difficulté les deux pièces pour réaliser la connexion, tout comme elle permet de voir la commande 5 et de contrôler le bon fonctionnement en cours d'intervention. De son côté, la minceur du film et sa souplesse font que la housse ne gêne en aucune manière la précision des gestes du praticien.

Les figures 1b et 2b montrent une autre forme d'exécution du dispositif selon l'invention qui ne se distingue du précédent que par ses proportions. Les mêmes références qu'aux figures 1a-2a, mais suivies de la lettre b au lieu de la lettre a, désignent les mêmes parties que précédemment et on ne les décrira donc pas. Il s'agit cette fois d'un dispositif adapté à être interposé entre une unité d'aspiration dont le diamètre interne amont standard, de 11 mm, est susceptible de recevoir une canule de même diamètre externe.

Les figures 1c et 2c montrent une autre forme d'exécution de l'invention qui se distingue des précédentes par le fait que le canal 11c de son adaptateur 8c ne comporte pas d'épaulement interne. Ce dispositif est destiné à permettre le montage, sur un embout 1, d'une canule dont le diamètre externe standardisé (7 mm) est plus petit que le diamètre interne (11 mm) de l'extrémité amont 2 dudit embout.

On pourrait également obtenir cet effet de réduction de diamètre, entre canule et embout, en enfilant la canule de plus petit diamètre dans l'adaptateur 8b des figures 1b-2b, jusque dans la zone E de celui-ci.

## Revendications

**1** **-** Dispositif jetable pour la protection d'une unité d'aspiration chirurgicale du type comprenant un conduit d'aspiration (4) ayant une extrémité amont et une extrémité aval, par référence au sens d'écoulement (F) sous l'effet de l'aspiration, ladite extrémité amont comportant un embout (1) adapté à recevoir une canule d'aspiration (7) et ladite extrémité aval étant susceptible d'être reliée à une source de vide, caractérisé en ce qu'il comprend :
- d'une part, un adaptateur (8, 8a-c) présentant une extrémité amont (9) et une extrémité aval (10̸) mises en communication par un canal traversant (11a-c) , ladite extrémité amont (9) de l'adaptateur (8, 8a-c) étant adaptée à être connectée avec ladite canule (7), tandis que son extrémité aval (10̸) est adaptée à être connectée avec l'extrémité amont (2) dudit embout (1) et,
- d'autre part, une housse tubulaire (16) dont une extrémité est solidarisée audit adaptateur (8, 8a-c) et dont l'autre extrémité est libre, ladite housse définissant une cavité adaptée à recevoir ledit embout (1) et l'extrémité amont dudit conduit (4) lorsque ledit embout (1) est connecté audit adaptateur (8, 8a-c).

**2** **-** Dispositif selon la revendication 1, caractérisé en ce que ledit adaptateur (8, 8a-8c) est connectable à ladite canule (7) par réception de l'extrémité de ladite canule dans l'extrémité amont dudit canal (11a-c).

**3** **-** Dispositif selon la revendication 1 ou 2, caractérisé en ce que ledit adaptateur (8, 8a-c) est connectable audit embout (1) par réception de son extrémité aval dans l'extrémité amont (2) dudit conduit.

**4** **-** Dispositif selon l'une quelconque des revendications 1 à 3, caractérisé en ce que ledit adaptateur (8, 8a,b) et son canal traversant (11a,b) sont de section circulaire et en ce que le diamètre interne (DiC) de l'extrémité amont dudit canal traversant (11a,b) est égal au diamètre externe aval (DeE) dudit adaptateur.

**5** **-** Dispositif selon l'une quelconque des revendications 1 à 4, caractérisé en ce que l'extrémité aval dudit canal (11a-c) est munie d'un clapet anti-retour (13).

**6** **-** Dispositif selon la revendication 5, caractérisé en ce que ledit clapet est un clapet souple (13) à lèvres normalement jointives mais adaptées à s'écarter sous l'effet d'une aspiration.

**7** **-** Dispositif selon la revendication 6, caractérisé en ce que ledit clapet est un clapet souple à lèvres en croix.

**8** **-** Dispositif selon l'une quelconque des revendications 1 à 7, caractérisé en ce que ledit adaptateur (8, 8a-c) est en matière plastique moulée.

**9** **-** Dispositif selon l'une quelconque des revendications 1 à 8, caractérisé en ce que ladite housse (16) est en un film de matière plastique transparent.

**10̸** **-** Ensemble de protection comprenant un dispositif (6) selon l'une quelconque des revendications 1 à 9 conditionné dans un emballage étanche et au contenu stérilisé.
